# EUROPEAN PATENT APPLICATION

(11) **EP 3 406 590 A1**
(43) Date of publication of application: **28.11.2018**
(21) Application number: 18174421.0
(22) Date of filing: 25.05.2018
(51) Int. Cl.: C07C 229/18, C05D 9/02, C07C 229/36, C07C 229/76

(54) **CHELATING AGENTS FOR TREATING NUTRITIONAL DISORDERS OF PLANTS**

(30) Priority: 26.05.2017 IT 201700057247
(71) Applicant: Valagro S.p.A., 66041 Atessa (Chieti) (IT)
(72) Inventor: BIASONE, Alessandro, 65123 PESCARA (IT)
(74) Representative: Biggi, Cristina

(57) **Abstract**

The present disclosure relates to a compound of general formula (I): 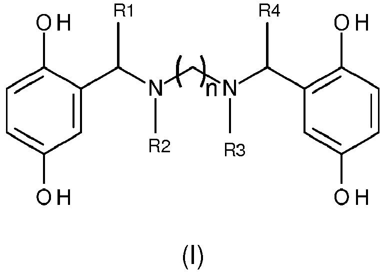 wherein R1 and R4 are independently selected from H and COOH, R2 and R3 are independently selected from H and (CH₂)ₘCOOH, in which m = 1, 2 or 3, and n is equal to 2 or 3.

The present disclosure also relates to a chelate compound, which is a complex of the compound of formula (I) with a metal ion selected from the group consisting of Fe, Cu, Mg, Ca, Mn and Zn.

The invention regards also the use of the compounds of formula (I) and/or their chelate complex for treating and/or preventing nutritional disorders of plants, in particular for treating and/or preventing iron chlorosis of plants.

## Description

### TECHNICAL FIELD

The present disclosure relates to hydroquinone-based polyamino carboxylic acids, in particular to ethylenediamine-N,N'-bis(*o*-hydroxyphenyl acetic)acid (*o*,*o*-EDDHA) and N,N'-bis(2-hydroxybezyl)ethylenediamine-N,N'-diacetic acid (HBED) analogues, and their use for treating and/or preventing nutritional disorders of plants, in particular for treating and/or preventing iron chlorosis of plants.

### BACKGROUND ART

Nowadays, fertilization with synthetic Fe³⁺ chelates is the most common agricultural practice against iron chlorosis of plants growing in calcareous and/or alkaline soils. Fe-o,o-EDDHA is the best known chelate compound employed to this aim; nevertheless during the preparation of this chelating agent, currently based on Mannich-like reaction, involving a phenol (or a substituted phenol), ethylenediamine and glyoxylic acid, the formation of by-products occurs. The presence of the polycondensation by-products *o*,*p-*EDDHA and *p*,*p*-EDDHA isomers, which display a lower stability constant with iron(III), reduces the effectiveness of the final fertilizer composition. Alternative synthetic procedures of FeEDDHA were proposed by Petree et al. in the patent publication US 4,130,582, wherein a method able to yield compounds with better chelating capabilities, consisting in employing an excess phenol as solvent system, is disclosed. This approach unfortunately has the great disadvantage that a large excess of phenol is required (up to 13 fold) to limit the unwanted *o*,*p*-EDDHA and *p*,*p*-EDDHA isomers, thus leading to a reaction mixture unsuitable for the final chelation step with an Fe(II/III) salt. Up to now, this problem has only been bypassed by introducing an additional extraction step of phenol excess from the reaction mixture before the addition of the Fe(II/III) salt, as reported in US 4,130,582. The suitable starting phenols are described by a general formula having two substituents, with undefined position, independently selected from hydrogen, methyl, ethyl, halogen, methoxy, hydroxyl, carboxyl, sulfo and acetyl.
Other chelating agents with substituted phenols were proposed for example by Fernandez et al. in J. Agric. Food Chem. 2002, 50, 284-290, who describe p-phenolsulfonic and hydoxybenzencarboxylic acid based chelates. In this case, an aqueous solution and an almost stoichiometric amount of p-phenolsulfonic and p-hydroxybenzoic acids is employed, but the polycondensation products and the unreacted starting reagents are not selectively removable from the reaction mixture, thus leading again to a less effective final fertilizer (J. Agric. Food Chem. 2002, 50, 284-290; Journal of Chromatography A, 1064 (2005) 67-74).
Recently, N,N'-bis(2-hydroxybezyl)ethylenediamine-N,N'-diacetic acid (HBED) has become a promising alternative to *o*,*o*-EDDHA and their derivatives, thanks to a higher stability constant for Fe³⁺ with respect to *o*,*o*-EDDHA; the stability constant is indeed 4 orders of magnitude greater than EDDHA (10³⁹ vs 10³⁵), as reported by Rayo et al. in J. Agric. Food Chem. 2009, 57, 8504-8513. Nevertheless, even if this chelating compound can be obtained with a high purity, the synthesis process requires very long reaction times, the use of the expensive 2-hydroxybenzaldehyde and two catalytic hydrogenation steps (as taught by Nawrocki et al. in EP 2 039 679). In EP 2 039 679 a method for the preparation of HBED and its derivatives having a substituent selected from H, C₁-C₄ alkyl, CH₂OH, SO₃M and COOM (with M being H, Na, K or NH₄) in the para position of the phenolic ring is disclosed. The method consists of a first step wherein a salen compound is obtained by reacting ethylenediamine with a bisubstituted phenol. Thereafter the double bonds of the obtained salen compound are reduced and finally a reductive amination of glyoxylic acid with the reduced salen substituted compound is carried out.
Therefore, there is a strong need for alternative metal ion chelating compounds, useful for the treatment of nutritional disorders, in particular iron chlorosis of plants. The new metal ion chelating compounds should also be synthesizable with a simple process that limits the formation of polycondensation and isomer by-products.
The biodegradation of chelating compounds is becoming a relevant environmental issue. The persistence of recalcitrant chelating compounds in the environment could cause the leaching of heavy metals from calcareous soils to the groundwater (J Hazard Mater. 2007, 147(3):768-75; Agriculture, Ecosystems & Environment 102 (3), 307-318). The recent trend is to use biodegradable chelating agents such as (S,S)-EDDS to prevent heavy metals leachability. EDDHA and HBED are mostly used in agriculture and are considered not readily biodegradable.

### SUMMARY OF THE DISCLOSURE

The present disclosure relates to compounds of formula (I), which are analogues of EDDHA or HBED. The compounds of formula (I) are hydroquinone-based analogues of EDDHA or HBED, having high effectiveness as chelating agents for metal ions, preferably for Fe ions. The compounds of the disclosure facilitate plant assimilation of iron ions due to the ability of the hydroquinone residue to affect iron reduction from Fe³⁺ to Fe²⁺. Fe(II) is better assimilated by the plants than Fe(III) ions (Plant Physiology 71:949-954; Journal of Biological Chemistry 268: 20 958-20 965; Plant & Cell Physiology 52: 2173-2189; Plant, Cell & Environment 35: 1171-1184; Plant and Soil 329: 1-25; New Phytologist (2014) 201: 155-167). Hydroquinone has a lower oxidation potential with respect to a phenolic group and thus reduces metals more easily than phenol (J.of Electroanalytical Chemistry (2011), 655, 9-16). In addition, its oxidation is reversible. Furthermore, the compounds of the disclosure possess improved biodegradability and enhanced mobility in the soil due to the high polar nature of the hydroxyquinone residue and fungi and bacteria are capable of transforming hydroquinone (BioMed Research International Volume (2013) Article ID 542168).
Therefore, the disclosure refers also to a chelate compound obtained from the complexation reaction of a compound of formula (I) with a metal ion selected from the group consisting of Fe, Cu, Mg, Ca, Mn and Zn, preferably Fe.
The chelate compound is used for the treatment or prevention of a nutritional disorder of a plant, in particular for the treatment of iron chlorosis of a plant. Preferably, the plant is a row crop (e.g. soybean), orchard crops (e.g. pear), horticultural crops, (e.g. rocket).
The disclosure refers also to a process for the preparation of the compounds of formula (I) characterized by a limited number of reaction steps and few by-products. The use of the starting material hydroquinone in place of the prior art phenol allows to produce a final product having a higher yield of ortho-ortho fraction using far less starting material. This is an advantage in terms of costs of the production process.

### DETAILED DESCRIPTION OF THE DISCLOSURE

In a first aspect, the present disclosure relates to a compound of general formula (I): 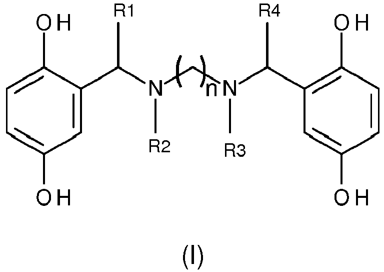 wherein R1 and R4 are independently selected from H and COOH, R2 and R3 are independently selected from H and (CH₂)ₘCOOH, in which m = 1, 2 or 3, and n is equal to 2 or 3.
In a second aspect, the present disclosure relates to a chelate compound, which is a complex of the compound of formula (I) with a metal ion selected from the group consisting of Fe, Cu, Mg, Ca, Mn and Zn.
In another embodiment of formula (I), R1 and R4 are independently selected from H and COOH, R2 and R3 are independently selected from H and (CH₂)ₘCOOH, in which m = 1 or 2, and n is equal to 2.
In another embodiment of formula (I), R1 and R4 are independently selected from H and COOH, R2 and R3 are independently selected from H and CH₂COOH and n is equal to 2.
In another embodiment of formula (I), R1 and R4 are both COOH and R2 and R3 are both H and n is 2 or 3.

In another embodiment of formula (I), R1 and R4 are both H and R2 and R3 are both (CH₂)ₘCOOH, in which m = 1, 2 or 3, and n is equal to 2 or 3.
In another embodiment of formula (I), R1 and R4 are both H and R2 and R3 are both CH₂COOH and n is equal to 2.

The above embodiments refers also to the chelate compound which is a complex of the compound of formula (I) with a metal ion selected from the group consisting of Fe, Cu, Mg, Ca, Mn and Zn
In a preferred embodiment, a compound of formula (I) is ethylenediamine-N-N'-bis(2,5-dihydroxyphenylacetic) (EDDDHA), which corresponds to the general formula (I) in which R1 is COOH, R2 and R3 are H, R4 is COOH and n=2, and has the following formula:

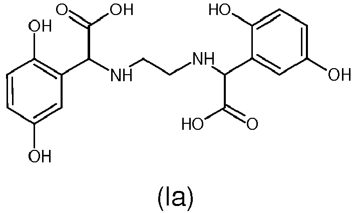

In a preferred embodiment, the compound of formula (I) is N,N'-bis-(2,5-dihydroxyphenyl))-ethylenediamine-N,N'-diacetic acid (DHBED), which corresponds to the general formula (I) in which R1, R4 are H, R2 and R3 are CH₂COOH, n=2 and has the following formula:

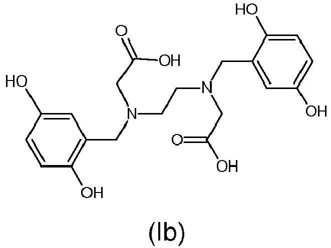

Compound la can be considered analogue of EDDHA, while compound Ib can be considered analogue of HBED.

If the synthesis reaction to make the compounds of formula (I) is carried out using hydroquinone as limiting reagent, a final mixture of a compound of formula (I) as described above and polycondensation products thereof is formed. The polycondesation products have the following general formula (II): 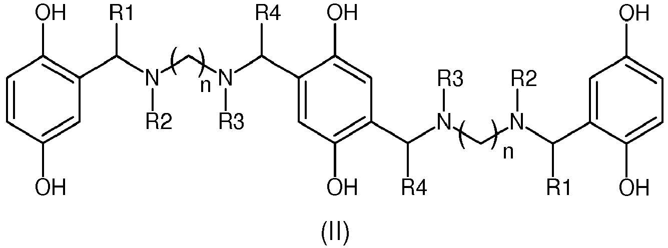 wherein R1 and R4 are independently selected from H and COOH, R2 and R3 are independently selected from H and (CH₂)ₘCOOH, in which m = 1, 2 or 3, and n is equal to 2 or 3.
In another embodiment of formula (II), R1 and R4 are independently selected from H and COOH, R2 and R3 are independently selected from H and (CH₂)ₘCOOH, in which m = 1 or 2, and n is equal to 2.
In another embodiment of formula (II), R1 and R4 are independently selected from H and COOH, R2 and R3 are independently selected from H and CH₂COOH and n is equal to 2.
In another embodiment of formula (II), R1 and R4 are both COOH and R2 and R3 are both H and n is 2 or 3.
In another embodiment of formula (II), R1 and R4 are both H and R2 and R3 are both (CH₂)ₘCOOH, in which m = 1, 2 or 3, and n is equal to 2 or 3.
In another embodiment of formula (II), R1 and R4 are both H and R2 and R3 are both CH₂COOH and n is equal to 2.

The disclosure thus relates to the compound of formula (II), as above described, and to a mixture of a compound of formula (II) and a compound of formula (I).

In a preferred embodiment, the disclosure relates also to a polycondensation product (IIa): 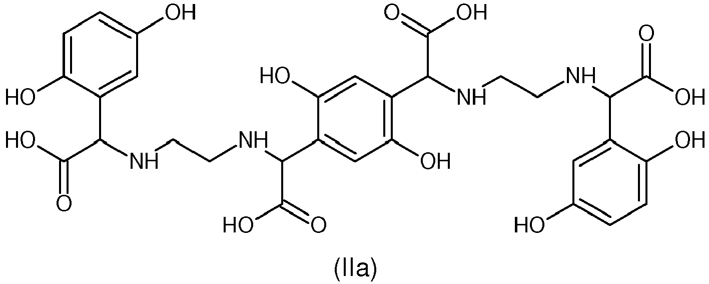 and to a mixture of (IIa) and compound (Ia).

In another preferred embodiment, the disclosure relates to a polycondensation product (IIb): 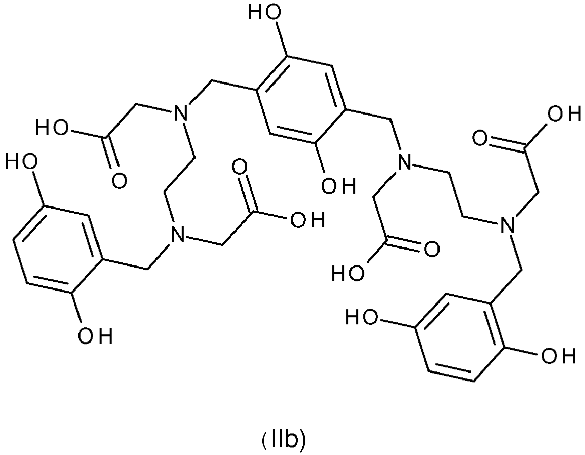 and to a mixture of (IIb) and compound (Ib).

With respect to the known compounds EDDHA, EDDHSA, EDDCHA, EDDHMA, that are respectively synthetized starting from phenol, p-hydroxybenzensulfonic acid, p-hydroxybenzoic acid, p-cresol, the use of hydroquinone for the preparation of the compounds of formula (I), according to the present disclosure, leads to the formation of polycondensation products of formula (II). The polycondensation products (II) are bis-exadentate chelating agents, which can bind two iron (III) ions in a stable way, thus contributing to the overall effectiveness of the mixture.
Thus, the present disclosure relates also to a chelate compound, which is a complex of the compound of formula (II) with two metal ions selected from the group consisting of Fe, Cu, Mg, Ca, Mn and Zn. The disclosure relates also to a mixture of a chelate of formula (I) and a chelate of formula (II). Preferably, the chelate compound is a complex between (IIa) or (IIb) and two metal ions selected from the group consisting of Fe, Cu, Mg, Ca, Mn and Zn.

The present disclosure relates also to the use of a compound of formula (I) and/or formula (II) as a chelating agent for a metal ion selected from the group consisting of Fe, Cu, Mg, Ca, Mn and Zn. Thus, a compound of formula (I) and/or formula (II) and a complex with either of Fe, Cu, Mg, Ca, Mn or Zn ion (i.e. a chelate compound) is used to treat or prevent nutritional disorders of a plant. For example, a compound of formula (I) and/or formula (II) and a complex thereof can be used to treat row crops (e.g. soybean), orchard crops (e.g. pear), horticultural crops, (e.g. rocket). ....
When a compound of formula (I) and/or formula (II) is complexed with Fe ions, the resulting iron chelate can be used to treat and prevent iron chlorosis of a plant. For example, a compound of formula (I) and/or formula (II) and a complex thereof with Fe is used to treat and prevent iron chlorosis on row crops (e.g. soybean), orchard crops (e.g. pear), horticultural crops, (e.g. rocket).
The disclosure relates also to a method for treating and/or preventing nutritional disorders of a plant which comprises the step of applying a complex of a compound of formula (I) and/or formula (II) with a metal ion selected from Fe, Cu, Mg, Ca, Mn and Zn (i.e. a chelate compound) to the plant, wherein the plant is preferably row crops (e.g. soybean), orchard crops (e.g. pear), horticultural crops, (e.g. rocket). The disclosure relates also to a method for treating and/or preventing iron chlorosis of a plant which comprises the step of applying a complex of a compound of formula (I) and/or formula (II) with Fe(II) or Fe(III) ions (i.e. a iron chelate) to the plant, wherein the plant is preferably row crops (e.g. soybean), orchard crops (e.g. pear), horticultural crops, (e.g. rocket).

The present disclosure relates also to a process for the preparation of the compounds of formula (I) and formula (II).
The applicant surprisingly found that the use of hydroquinone as a starting material in place of the prior art phenol improves the synthetic process, in terms of selectivity for the desired compounds, by producing higher yield of a compound of formula la and consequently less by-products. Moreover, the applicant found that the disclosed process increases productivity because of the use of far less amount of phenolic starting material in comparison, for example, to known EDDHA synthetic process. For example the typical ratios of phenol/ethylendiamine are 13 for EDDHA and 1.5 for compounds of formula (I) where n=2; R2 and R3=H; R1 and R4=COOH.
Furthermore, the presence of a hydroquinone residue in the chemical structure brings advantages to the biological activity of the compounds (as explained above). but also in terms of biodegradability of the chelate compound in the environment. The compounds of formula (I) and their chelates include a hydroquinone residue which renders them more easily degradable by environmental microorganisms (e.g. fungi) with respect to the known chelates having a phenol residue, due to the presence of 2 hydroxyl groups in the hydroquinone (instead of just one hydroxyl group as in a phenol residue).

The synthesis process of EDDHA analogues of formula (I) and formula (II) (for example compound Ia- EDDDHA - and IIa - polycondesation product) is simpler and more selective than the prior art process of preparation of EDDHA. In the prior art a phenol group is used as starting material. The hydroxyl group of the phenol orients the Mannich reaction to the ortho and para positions, thus generating ortho isomers or para isomers respectively. From a statistical point of view the probability is 66% for the ortho position and 33% for the para position. Other factors such as temperature, solvent and pH can change the o/p ratio.
The use of hydroquinone exclude the reaction at para position since such position is occupied by the second hydroxyl group. As a consequence the reaction at ortho position is the only one allowed.
If the reaction is carried out using hydroquinone as limiting reagent, a final mixture enriched in polycondensation products, such as compound (IIa), is formed.

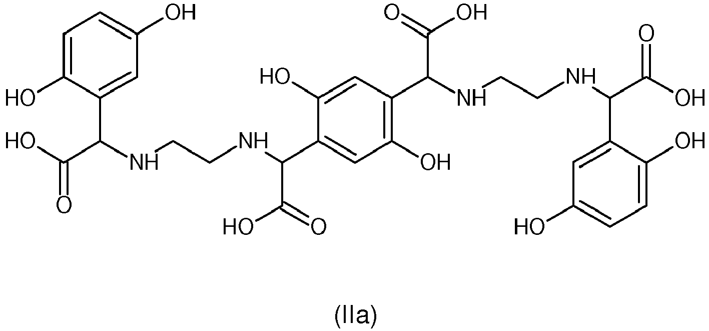

The chemical synthesis of the EDDHA analogues of formula (I) (such as EDDDHA) is carried out in a single step (see for example Scheme 2 below) in aqueous solvent with an almost stoichiometric or a small excess of hydroquinone. The unreacted hydroquinone can be removed by a liquid-liquid extraction step with an organic solvent. Because the excess of hydroquinone is much smaller than that of phenol involved in the prior art EDDHA synthesis, the volume of organic solvent used is smaller.
The reaction time of the EDDHA analogues of formula (I) is less than one half of the EDDHA typical reaction time.
The chelate compound is formed by adding a metal ion salt to the aqueous phase containing the EDDHA analogue of formula (I). For example, the iron ion salts preferably used are iron(II)sulfate, iron(III)sulfate, iron(III)nitrate, iron(II)chloride, iron(III)chloride and their different hydrated forms.

As mentioned before, the synthesis process of the EDDHA analogues of formula (I) comprises a single step in which glyoxylic acid, a strong base (such as KOH or NaOH), an alkyl(C2-C3)diamine and hydroquinone are mixed together in an aqueous solvent. The temperature is raised to 70-90°C and the reaction mixture is kept under stirring for 2-4 hours.
At the end of the reaction, the excess of hydroquinone is removed with an extraction in a suitable organic solvent.
The synthesis process of a chelate compound includes a further step in which a metal ion salt, such as an iron(II) or iron(III) salt, is added to the aqueous purified solution of the EDDHA analogue of formula (I).

The synthesis process of the HBED analogues of formula (I) (e.g. compound Ib - DHBED) is less expensive and simpler than the synthesis process of the known HBED.
The chemical synthesis of HBED requires very long reaction times, the use of the expensive salicylaldehyde and two catalytic hydrogenation steps (see Scheme 1 below).

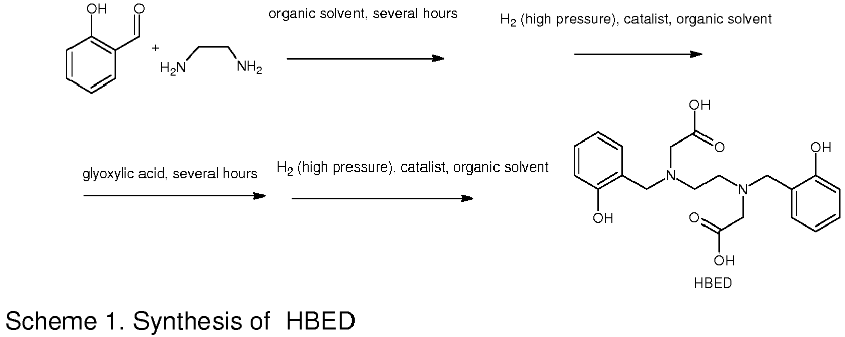

The use of hydroquinone to produce the HBED analogues of formula (I) allows to avoid the use of a very expensive 2-hydroxybenzaldehyde and also allows to avoid one hydrogenation step.
The process for the preparation of the HBED analogues of formula (I) (Scheme 3) comprises two steps in which formaldehyde, hydroquinone and alkyl(C2-C3)diamine react in a polar solvent consisting on C1-C3 alkanol/water mixture. The temperature is raised to 70-90°C and the reaction mixture is kept under stirring for 2-4 hours. At the end of the reaction a strong base and glyoxylic acid were added and the mixture was let to stand at 50°C for 1-3 hours. Reductive amination was carried out for 1-48 hours after the addition of a catalyst (preferably Ni Raney or Pd/C).
At the end of the reaction, the excess of hydroquinone is removed with an extraction in a suitable organic solvent.
The synthesis process of a chelate compound includes a further step in which a metal ion salt, such as an iron(II) or iron(III) salt, is added to the aqueous purified solution of the HBED analogues of formula (I).

Furthermore the synthesis process of the HBED analogues of formula (Ib) (Scheme 4) can be accomplished by a single step reaction mixing EDDA (N, N'-ethylenediaminediacetic acid), formaldehyde, a strong base (NaOH or KOH) and hydroquinone in aqueous solvent. The temperature is raised to 70-90°C and the reaction mixture is kept under stirring for 2-4 hours.
At the end of the reaction, the excess of hydroquinone is removed with an extraction in a suitable organic solvent.
The synthesis process of a chelate compound includes a further step in which a metal ion salt, such as an iron(II) or iron(III) salt, is added to the aqueous purified solution of the HBED analogues of formula (I).

### EXAMPLES

### Typical procedure for the preparation of EDDDHA (formula Ia)

Glyoxylic acid (1mol) was dissolved in 450mL of H₂O. To this solution NaOH 50% (0.6 mol) was added drop wise under stirring and let to stand for 5 minutes. Then Ethylendiamine (0.5 mol) was poured slowly under immersion to avoid fume development (hexotermic reaction). Hydroquinone (1.5 mol) was added to the final mixture and the temperature is raised to 80°C for 3 hours.
At the end of the reaction the mixture was cooled down and the excess of hydroquinone was extracted with a suitable organic solvent (e.g. i-Butyl Acetate).
To the aqueous phase an iron (II/III) salt was added to achieve the final chelate (Fe-EDDDHA), according to the following scheme:

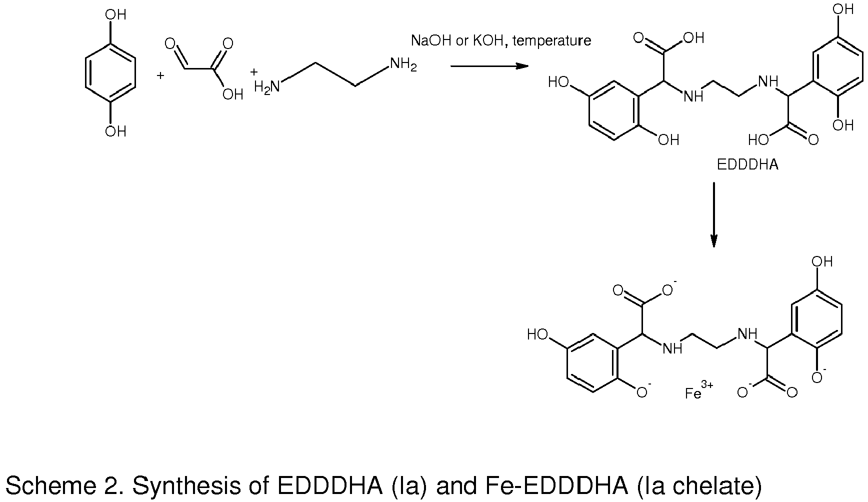

The mixture of *rac-* and *meso*-EDDDHA (ratio ¼) was obtained as pink solid by precipitation at pH 6 with HCl 1M.
meso-EDDDHA: ¹H NMR (500 MHz, D₂O: NaOD) δ 2,70-2,85(m, 2H), 2,70-2,98 (m, 2H), 4,39 (s, 1H), 6,68-6,75 (m, 6H, ArH).
¹³C NMR (700 MHz) δ 47.77, 66.73, 119.10, 119.21, 120.32, 127.12, 151.54, 151.88, 179.81.
FT-IR (ATR) cm⁻¹ 3485, 3060, 2725, 1610, 1461, 1375, 1208.
ESI-MS 393,1293 [M+H⁺]⁺, 347,1235 [M+H⁺-CO₂]⁺, 283,0915 [M+H⁺-Hydroquinone]⁺.
*rac*-ED3HA: ¹H NMR (500 MHz, D₂O: NaOD) δ 2,70-2,85(m, 2H), 2,70-2,98 (m, 2H), 4,34 (s, 1H), 6,68-6,75 (m, 6H, ArH).
¹³C NMR δ (700 MHz) 47.51, 66.78, 119.27, 119.27, 120.27, 127.03, 151.22, 151.91, 179.62.
FT-IR (ATR) cm⁻¹ 3485, 3060, 2725, 1610, 1461, 1375, 1208.
ESI-MS 393,1293 [M+H⁺]⁺, 347,1235 [M+H⁺-H₂CO₂]⁺, 283,0915 [M+H⁺-Hydroquinone]⁺.

### Typical procedure for the preparation of DHBED

EDDA (1mol) was dissolved in 750mL of H₂O. To this solution NaOH 50% (1 mol) was added drop wise under stirring and let stand for 5 minutes. Formaldehyde 37% (2 mol) was added dropwise under immersion and the solution was let under stirring for 30 min. Hydroquinone (2.2 mol) was added to the final mixture and the temperature is raised to 80°C for 3 hours.
At the end of the reaction the mixture was cooled down and the excess of hydroquinone was extracted with a suitable organic solvent (e.g. i-Butyl Acetate).
To the aqueous phase an iron (II/III) salt was added to achieve the final chelate (Fe-DHBED).

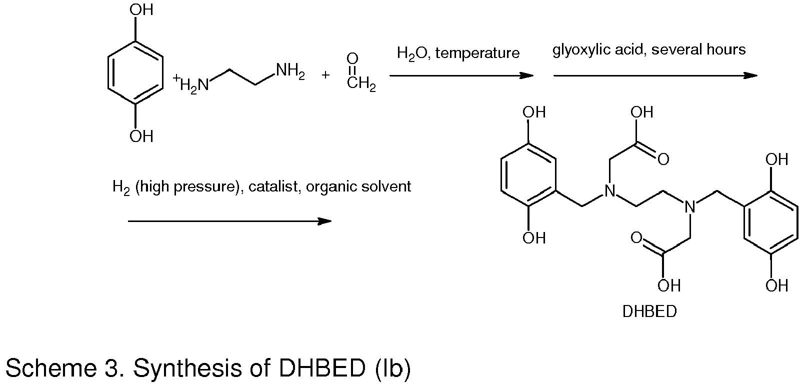

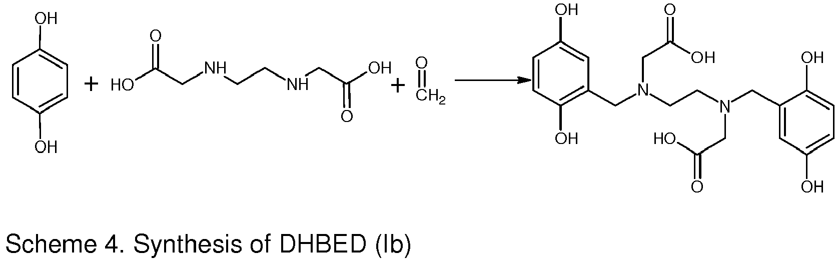

DHBED was obtained as pink solid by precipitation at pH 6 with HCl 1M. ¹H NMR (500 MHz, D₂O: NaOD) δ 2,80(m, 2H), 3,19 (s, 2H), 3,70 (s, 2H), 6,60-6,78 (6ArH).
¹³C NMR (700 MHz) δ 52,0, 58.57, 59.8, 119.2, 119.7, 120.0, 125.5, 151.3, 152.1, 179.8.
FT-IR (ATR) cm⁻¹ 3187, 3030, 2970, 2833, 1625, 1387, 1270. ESI-MS 421,1612 [M+H⁺]⁺, 299,1243 [M+H⁺-C₇H₆O₂]⁺.

### Greenhouse tests

### Agronomic trials on horticultural crop: rocket

The following iron chelates were assessed in greenhouse trials :
- FeEDDHA, total Fe 6%, 4.8% Fe chelated with o,o-EDDHA
- FeHBED, total Fe 6%, 6% Fe chelated with HBED
- Fe EDDDHA prot 13, total Fe 7,5%, roughly 6-6.5% of iron chelated with EDDDHA and polycondensation products
- Fe EDDDHA prot 14, total Fe 7 %, roughly 6% of iron chelated with EDDDHA and polycondensation products
The rocket was used as model plant to evaluate iron chelates effectiveness. The plants were grown in a calcareous soil to induce chlorosis (checked measuring the Spad values of leaves). A homogeneous group of plants was selected for the treatment in order to have similar Spad values at time 0. 20 plant per thesis were considered.

The experiment lasted 14 days after the treatment with the solutions of chelates. The Spad values were measured at day 0 (T0), 3 (T1), 5 (T2), 7 (T3), 10 (T5), 14 (T5).
Three levels of concentration were used to assess the effectiveness of chelates, respectively 0.1, 0.5 and 1 ppm of chelated iron/Kg of soil (Table 1).
The working solutions were applied once at Time 0.
20 mL of the freshly prepared iron chelate solution was applied under the surface of the soil with a syringe to prevent photo-degradation of chelates:

**Table 1: the doses (mg) of Fe chelates/Kg of soil applied corresponding to 0.1, 0.5, and 1 mg of Fe chelated per Kg of soil.**

| NAME | 0.1 ppm Fe/Kg soil | 0.5 ppm Fe/Kg soil | 1 ppm Fe/Kg soil |
|---|---|---|---|
| Fe EDDHA | 0,35 | 1,77 | 3,54 |
| Fe HBED (Bolikel) | 0,28 | 1,42 | 2,83 |
| Fe EDDDHA prot 13 | 0,28 | 1,42 | 2,83 |
| Fe EDDDHA prot 14 | 0,28 | 1,42 | 2,83 |

### Determination of ΔSpad index.

The average values of ΔSpad index were reported in Tables 2-4.

**Table 2. ΔSpad averaged values of rocket leaves measured at different times in trial at 0.1 mg of chelated Iron per Kg of soil._Different letters in the same column denote significant differences among treatments for Newman-Keuls test.**

| | T1 | | T2 | | T3 | | T4 | | T5 | |
|---|---|---|---|---|---|---|---|---|---|---|
| UTC | -1,1 | c | -1,2 | d | -1,4 | e | -1,4 | d | 0,6 | d |
| FeHBED | 3,2 | ab | 5,6 | bc | 7,0 | bc | 8,1 | b | 9,7 | b |
| FeEDDHA | 4,3 | ab | 8,3 | a | 9,9 | a | 11,7 | a | 12,7 | a |
| Fe EDDDHA prot 13 | 4,0 | ab | 7,3 | ab | 8,4 | ab | 9,4 | ab | 10,7 | b |
| Fe EDDDHA prot 14 | 4,7 | a | 8,3 | a | 10,1 | a | 11,1 | a | 12,9 | a |

**Table 3. ΔSpad averaged values of rocket leaves measured at different times in trial at 0.5 mg of chelated Iron per Kg of soil. Different letters in the same column denote significant differences among treatments for Newman-Keuls test.**

| | T1 | | T2 | | T3 | | T4 | | T5 | |
|---|---|---|---|---|---|---|---|---|---|---|
| UTC | -1,1 | b | -1,2 | b | -1,4 | b | -1,4 | b | 0,6 | c |
| FeHBED | 2,8 | a | 5,2 | a | 6,6 | a | 7,5 | a | 11,2 | a |
| FeEDDHA | 3,2 | a | 5,1 | a | 6,3 | a | 7,5 | a | 9,7 | ab |
| Fe EDDDHA prot 13 | 2,0 | a | 3,6 | a | 4,8 | a | 5,8 | a | 7,7 | b |
| Fe EDDDHA prot 14 | 2,5 | a | 4,3 | a | 5,5 | a | 6,5 | a | 9,0 | ab |

**Table 4. ΔSpad averaged values of rocket leaves measured at different times in trial at 1 mg of chelated Iron per Kg of soil. Different letters in the same column denote significant differences among treatments for Newman-Keuls test.**

| | T1 | | T2 | | T3 | | T4 | | T5 | |
|---|---|---|---|---|---|---|---|---|---|---|
| UTC | -1,1 | b | -1,2 | b | -1,4 | b | -1,4 | b | 0,6 | b |
| FeHBED | 3,2 | a | 5,2 | a | 7,1 | a | 8,0 | a | 11,8 | a |
| FeEDDHA | 4,1 | a | 6,2 | a | 6,8 | a | 7,4 | a | 10,0 | a |
| Fe EDDDHA prot 13 | 3,4 | a | 6,0 | a | 7,0 | a | 7,8 | a | 10,1 | a |
| Fe EDDDHA prot 14 | 3,6 | a | 6,3 | a | 7,7 | a | 9,1 | a | 11,4 | a |

For untreated plants no increment of Spad Index was achieved. The data showed that both FeEDDDHA prototypes are able to increase the Spad Index also at the lower concentration.

### Field tests

### Agronomic trials on orchard crop: pear

Field trials were carried out in Emilia Romagna (Italy) on pear trees. In this area the treatment with iron chelates is a common practice to overcome chlorosis symptom.

Each experiment was carried out on 20 pear trees, 5 plots.
In the case of the control, no iron source was supplied to the trees.
The iron chelates were supplied to the plants the first time in the middle of April 2015 and the second time in the middle of May 2016.
The chelates used for the experiment were: FeEDDHA Valagro (6% total Iron, 4,8% Fe chelated with o,o-EDDHA isomers); FeHBED AKZO NOBEL (6% total Iron, 6% Fe chelated with HBED); the prototype FeEDDDHA-prot 13 (7,5% total Iron, 6-6,5% Fe chelated with EDDDHA isomers based on spectrophotometric determination using HPLC coupled to UV-Vis Diode Array detector). Because the significant fraction of Fe chelated with o,o-isomers, the following relative concentrations were applied to the soil:
FeEDDHA 20 Kg/ha
FeHBED 14 Kg/ha
FeEDDDHA prot 13 14 Kg/ha
The whole doses of the chelates were applied in two portions.
For each thesis, 20 plants in 4 blocks were used.

**Tables 5-9** show the evolution of Spad Index measured at different times after the first treatment.

**Table 5. Spad averaged values of pear leaves measured at different times. Different letters in the same column denote significant differences among treatments for Fisher's LSD test.**

| | T0 | | T1 | | T2 | | T3 | | T4 | | T5 | |
|---|---|---|---|---|---|---|---|---|---|---|---|---|
| days after the first application | 0 | | 8 | | 16 | | 28 | | 36 | | 43 | |
| UTC | 16,6 | a | 17,3 | a | 17,7 | a | 18,8 | b | 17,7 | c | 18,4 | b |
| FeEDDHA | 18,6 | a | 20,0 | a | 21,2 | a | 23,7 | a | 27,3 | a | 26,1 | a |
| FeHBED | 16,8 | a | 17,5 | a | 19,4 | a | 21,3 | ab | 23,5 | b | 24,3 | a |
| FeEDDDHA-prot 13 | 16,7 | a | 19,8 | a | 18,6 | a | 22,3 | a | 24,1 | ab | 25,7 | a |

**Table 6. Spad averaged values of pear leaves measured at different times. Different letters in the same column denote significant differences among treatments for Fisher's LSD test.**

| | T0 | | T1 | | T2 | | T3 | | T4 | | T5 | | T6 | |
|---|---|---|---|---|---|---|---|---|---|---|---|---|---|---|
| days after the first application | 0 | | 7 | | 12 | | 19 | | 27 | | 39 | | 48 | |
| UTC | 21,1 | a | 20,5 | b | 22,0 | b | 22,8 | b | 23,5 | c | 23,8 | c | 23,8 | c |
| FeEDDHA | 20,1 | a | 23,4 | a | 23,4 | ab | 25,7 | a | 30,5 | a | 32,4 | ab | 32,4 | a |
| FeHBED | 20,9 | a | 23,1 | a | 24,4 | ab | 26,2 | a | 28,7 | ab | 30,7 | ab | 30,7 | ab |
| FeEDDDHA-prot 13 | 21,8 | a | 24,2 | a | 25,3 | a | 26,4 | a | 27,2 | b | 35,6 | a | 35,6 | a |

**Table 7. Spad averaged values of pear leaves measured at different times. Different letters in the same column denote significant differences among treatments for Fisher's LSD test.**

| | T0 | | T1 | | T2 | | T3 | | T4 | | T5 | |
|---|---|---|---|---|---|---|---|---|---|---|---|---|
| days after the first application | 0 | | 7 | | 14 | | 27 | | 40 | | 48 | |
| UTC | 22,0 | ab | 24,8 | b | 23,9 | c | 24,9 | b | 23,6 | c | 21,4 | c |
| FeEDDHA | 23,1 | a | 27,6 | a | 28,1 | a | 30,2 | a | 32,8 | a | 37,2 | a |
| FeHBED | 22,8 | ab | 27,5 | a | 28,0 | a | 30,1 | a | 33,0 | a | 37,1 | a |
| FeEDDDHA-prot 13 | 22,1 | ab | 25,2 | b | 25,7 | b | 28,4 | a | 28,5 | b | 32,8 | b |

**Table 8. Spad averaged values of pear leaves measured at different times. Different letters in the same column denote significant differences among treatments for Fisher's LSD test.**

| | T0 | | T1 | | T2 | | T3 | | T4 | | T5 | | T6 | |
|---|---|---|---|---|---|---|---|---|---|---|---|---|---|---|
| days after the first application | 0 | | 6 | | 12 | | 19 | | 27 | | 38 | | 42 | |
| UTC | 20,7 | ab | 21,9 | b | 22,1 | b | 20,5 | c | 22,7 | b | 24,4 | b | 20,7 | c |
| FeEDDHA | 19,6 | b | 22,2 | b | 23,0 | b | 22,7 | b | 29,7 | a | 30,1 | a | 37,4 | a |
| FeHBED | 22,4 | a | 26,4 | a | 26,8 | a | 26,0 | a | 30,1 | a | 30,1 | a | 34,9 | ab |
| FeEDDDHA-prot 13 | 20,4 | ab | 23,0 | b | 24,1 | ab | 21,5 | b | 27,6 | a | 27,1 | ab | 32,9 | b |

**Table 9. Spad averaged values of pear leaves measured at different times. Different letters in the same column denote significant differences among treatments for Fisher's LSD test.**

| | T0 | | T1 | | T2 | | T3 | | T4 | |
|---|---|---|---|---|---|---|---|---|---|---|
| days after the first application | 0 | | 9 | | 21 | | 32 | | 44 | |
| UTC | 20,4 | a | 26,7 | b | 25,6 | c | 22,6 | c | 20,1 | d |
| FeEDDHA | 20,3 | a | 29,7 | a | 29,9 | a | 38,1 | a | 34,5 | a |
| FeHBED | 21,1 | a | 29,5 | a | 30,9 | a | 37,6 | a | 32,4 | b |
| FeEDDDHA-prot 13 | 20,2 | a | 30,1 | a | 30,7 | a | 38,4 | a | 32,5 | b |

In field trial the FeEDDDHA is able to relieve from chlorosis symptoms but there is a higher variability due to the different soil conditions of the tests.

## Claims

1. A compound of formula (I): 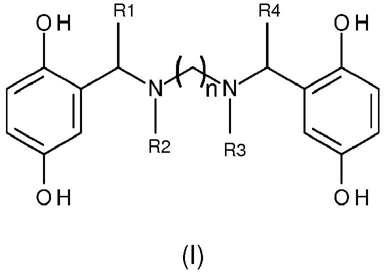 wherein R1 and R4 are independently selected from H and COOH, R2 and R3 are independently selected from H and (CH₂)ₘCOOH, in which m = 1, 2 or 3, preferably m = 1 or 2, more preferably m = 1, and n is equal to 2 or 3, preferably equal to 2.

2. The compound according to claim 1, in which R1 and R4 are both COOH and R2 and R3 are both H and n is 2 or 3.

3. The compound according to claim 1, in which R1 and R4 are both H and R2 and R3 are both (CH₂)ₘCOOH, in which m = 1, 2 or 3, preferably m = 1, and n is equal to 2 or 3, preferably equal to 2.

4. The compound of claim 1 having the following formulae:

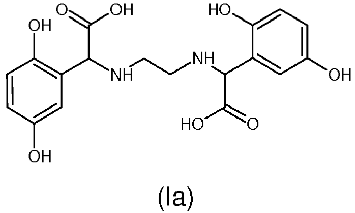

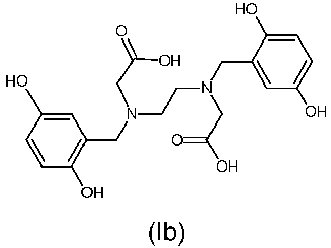

5. A polycondesation product having the following general formula (II): 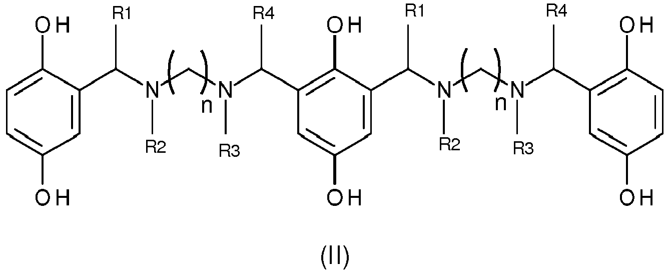 wherein R1 and R4 are independently selected from H and COOH, R2 and R3 are independently selected from H and (CH₂)ₘCOOH, in which m = 1, 2 or 3, preferably m = 1 or 2, more preferably m = 1 and n is equal to 2 or 3, preferably equal to 2.

6. The compound according to claim 5, in which R1 and R4 are both COOH and R2 and R3 are both H and n is 2 or 3.

7. The compound according to claim 5, in which R1 and R4 are both H and R2 and R3 are both (CH₂)ₘCOOH, in which m = 1, 2 or 3, preferably m = 1, and n is equal to 2 or 3, preferably equal to 2.

8. The compound according to claim 5, having formula (IIa) or (IIb):

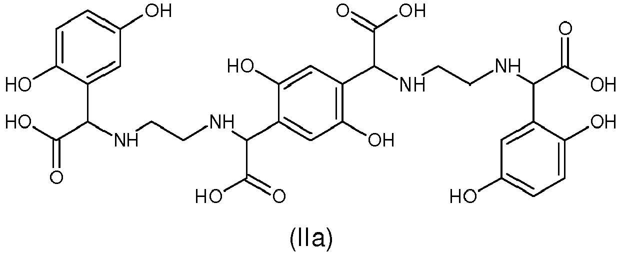

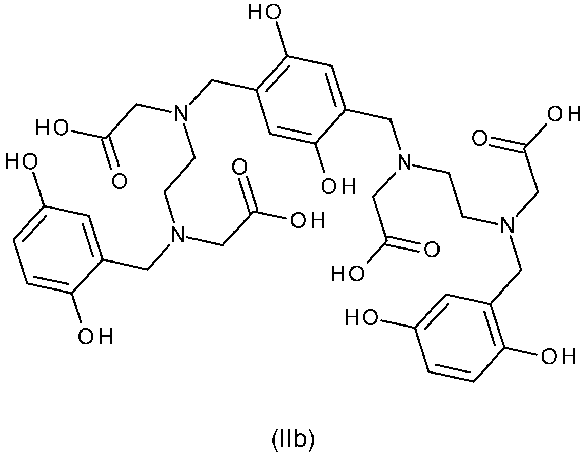

9. A mixture of a compound of formula (I) according to any claim from 1 to 4 with a compound of formula (II) according to any claim from 5 to 8.

10. A chelate compound which is a complex of the compound of formula (I) and/or formula (II) according to any claim from 1 to 4 and/or any claim from 5 to 8, with a metal ion selected from the group consisting of Fe, Cu, Mg, Ca, Mn and Zn, or with Fe(II) or Fe(III) ions.

11. A use of a compound of formula (I) and/or formula (II) according to any claim from 1 to 4 and/or any claim from 5 to 8, as a chelating agent for a metal ion selected from the group consisting of Fe, Cu, Mg, Ca, Mn and Zn.

12. A use of a chelate compound according to claim 10 for the treatment or prevention of a nutritional disorder of a plant or iron chlorosis of a plant.

13. The use according to claim 12, in which the plant is a row crop, preferably soybean, orchard crops, preferably pear, horticultural crops preferably rocket.

14. A process for the preparation of a compound of formula (I) according to any claim from 1 to 4 and/or a compound of formula (II) according to any claim from 5 to 8, in which hydroquinone is reacted, in a single step, with alkyl(C2-C3)diamine and formaldehyde or glyoxylic acid, in the presence of a strong base, wherein hydroquinone is used in an almost stoichiometric of small excess quantity.

15. A process for the preparation of a chelate compound of formula (I) and/or formula (II) according to claim 10 in which a compound of formula (I) and/or (II) is treated with a salt of a metal ion selected from the group consisting of Fe, Cu, Mg, Ca, Mn and Zn.
